# EUROPEAN PATENT APPLICATION

(11) **EP 4 481 754 A1**
(43) Date of publication of application: **25.12.2024**
(21) Application number: 23180251.3
(22) Date of filing: 20.06.2023
(51) Int. Cl.: G16H 20/40, G16H 20/10, A61M 16/00, A61B 5/00, G16H 40/63, G16H 70/40

(54) **SYSTEM AND METHOD FOR DETERMINING A CORRELATION BETWEEN MEDICATION INTAKE AND SLEEP DISORDERED BREATHING THERAPY OUTCOME**

(71) Applicant: Koninklijke Philips N.V., 5656 AG Eindhoven (NL)
(72) Inventor: HENDRIKS, Cornelis Petrus, Eindhoven (NL); KAHLERT, Joachim Johannes, Eindhoven (NL); MENA BENITO, Maria Estrella, 5656AG Eindhoven (NL); WESTERINK, Joanne Henriëtte Desirée Monique, Eindhoven (NL); VAN ZANTEN, Joyce, Eindhoven (NL); DOS SANTOS DA FONSECA, Pedro Miguel Ferreira, Eindhoven (NL); JUAN, Elsa, Eindhoven (NL); TSONEVA, Tsvetomira Kirova, Eindhoven (NL); VAN DEN ENDE, Daan Anton, Eindhoven (NL); WEIJSEN, Tim Elisabeth Joseph, Eindhoven (NL)
(74) Representative: Philips Intellectual Property & Standards

(57) **Abstract**

A system and method for determining a correlation between medication intake and sleep disordered breathing, SDB, therapy outcome for a subject taking at least one type of medication and being provided with SDB therapy. The SDB therapy comprises at least positive airway pressure, PAP, therapy provided by a PAP device. Medication information, relating to a medication intake of the subject, and SDB therapy information, relating to a therapy outcome of the SDB therapy, are obtained and correlated.

## Description

### FIELD OF THE INVENTION

The invention relates to the field of sleep disordered breathing therapy, and, in particular, to positive airway pressure (PAP) therapy.

### BACKGROUND OF THE INVENTION

Sleep-disordered breathing (SDB) conditions, such as obstructive sleep apnea (OSA) and central sleep apnea (CSA), are becoming increasingly common, and are particularly prevalent in older people, people with a high body mass index, smokers, heavy drinkers and people with conditions such as coronary artery disease, hypertension and diabetes mellitus.

SDB conditions are often treated using positive airway pressure (PAP) therapy, in which pressurized air is provided to a subject to keep the subject's airways open. Many subjects with SDB also take some form of medication, to alleviate their sleep disordered breathing and/or to treat one or more comorbidities.

For instance, some subjects may, in addition to undergoing PAP therapy, take medication to lower flow resistance in the upper airway (e.g. nasal decongestants or diuretics), to increase airway compliance in the lower airway (e.g. asthma medication and/or supplementary gases such as nitric oxide), and/or to induce sympathetic activity (e.g. medication that increases respiratory drive, shifts receptor thresholds, changes the loop gain, or increases muscle tone in the airways, tongue and/or respiration muscles). Some other types of medication, such as antidepressants, stimulants, weight loss drugs and hormones, may also have a positive effect on sleep disordered breathing.

Further, many types of medication that subjects with SDB may take to treat other conditions, such as barbiturates, benzodiazepines, beta-blockers, opioids, erectile dysfunction drugs, testosterone, and drugs causing weight gain, including their possible interactions with one another, may have a negative effect on sleep disordered breathing.

The interaction between various types of medication and PAP therapy, including the effect of drug-drug interactions on PAP therapy, is not well-understood, which means that clinicians risk adverse effects when prescribing medication to subjects undergoing PAP therapy, or prescribing PAP therapy to subjects already taking one or more types of medication. Even supposing a clinician is able to predict a risk for a prescribed medication (or a prescribed combination of medications), subjects do not always follow their prescription correctly. Further, many subjects take over-the-counter drugs in addition to their prescribed medication. These factors all present risks to subject safety.

### SUMMARY OF THE INVENTION

The invention is defined by the claims.

According to examples in accordance with an aspect of the invention, there is provided a processing system for determining a correlation between medication intake and sleep disordered breathing, SDB, therapy outcome for a subject taking at least one type of medication and being provided with SDB therapy comprising use of a positive airway pressure, PAP, device, the processing system being configured to: obtain medication information relating to a medication intake of the subject, wherein the medication information is indicative of at least a type of medication taken; obtain SDB therapy information relating to SDB therapy provided to the subject during at least one sleep session, wherein the SDB therapy information comprises at least one measure of therapy outcome; and process the medication information and the SDB therapy information to determine correlation data indicating a correlation between the medication data and the SDB therapy information.

By correlating medication information and SDB therapy information, interactions between the medication(s) taken by the subject and the therapy provided by the PAP device may be identified. This information may be used to make adjustments to the medication intake of the subject and/or to the PAP therapy provided to the subject to improve a therapy outcome and/or to reduce negative side effects on the subject.

The at least one type of medication taken by the subject may comprise medication for treating sleep disordered breathing (i.e. the SDB therapy may be combination therapy including PAP therapy and pharmacotherapy), and/or medication for treating one or more other conditions of the subject.

A therapy outcome may comprise a therapy efficacy and/or a therapy adherence.

In some examples, in response to the correlation data and/or the SDB therapy information satisfying a first predetermined condition, the processing system is further configured to control an output user interface to provide a user-perceptible output representative of the correlation data.

A user-perceptible output representative of the correlation data may, for example, comprise a correlation value or an indication that the correlation value exceeds a predetermined correlation threshold. The user-perceptible output may further comprise a measure of the strength of the correlation, a time lag of the correlation and/or an indication as to whether the correlation indicates a positive, negative or neutral effect on the subject.

This information may be provided to a clinician to allow the clinician to determine which, if any, adjustments should be made to the subject's medication and/or to the PAP therapy provided to the subject.

In some examples, the processing system is further configured to process the correlation data to determine a first recommended action.

The recommended action may, for instance, comprise an adjustment to the medication taken by the subject (e.g. a change of type, dosage and/or timing of medication) or an adjustment to the PAP therapy provided by the PAP device (e.g. a change in a pressure of air provided by the PAP device, a PAP therapy modality and/or a subject interface).

In some examples, the processing system is further configured to control an output user interface to provide a user-perceptible output of the first recommended action.

This provides a clinician with a suitable action to take in view of the correlation, while allowing the clinician to make a final decision on whether the recommended action is carried out.

In some examples, the first recommended action comprises an adjusted pressure to be provided by the PAP device, and the processing system is further configured to generate a control signal configured to control the PAP device to provide air to the subject at the adjusted pressure.

By directly controlling the PAP device to adjust the pressure of air provided to the subject, the subject's SDB therapy may be adjusted more quickly (e.g. during a sleep session of the subject). This also allows the SDB therapy to be adjusted responsive to day-to-day variations in medication intake (e.g. where the subject occasionally takes additional medication alongside their prescribed medication, or where the subject sometimes forgets to take prescribed medication).

In some examples, the processing system may control the PAP device to provide air at the adjusted pressure only if the adjusted pressure falls within a predefined pressure range, and may require clinician input for larger adjustments to the pressure.

In some examples, the processing system is further configured to: continue to obtain medication information and SDB therapy information for the subject after the first recommended action has been implemented; process the medication information and SDB therapy information to determine an effect of the first recommended action; and in response to the effect of the first recommended action satisfying a second predetermined condition, process the effect of the first recommended action to determine a second recommended action.

In other words, the processing system may monitor the effect of the first recommended action, and recommend an alternative or additional action if the first recommended action does not have a desired effect.

In some examples, the medication information further comprises a dosage and/or a timing of taking medication for at least one medication taken by the subject.

This allows a more detailed assessment of an interaction between the medication and the PAP therapy. For example, a different dosage of the same medication, or taking the same medication at a different time (relative to a time of sleep), may cause the medication to interact differently with the PAP therapy.

In some examples, the medication information is acquired by at least one of: a user input device, a sensor of a medication-dispensing device, a camera and/or a wearable biosensor.

The use of such devices may provide information about the medication actually taken by the subject.

Additionally or alternatively, prescription information for the subject may provide information about a type of medication that can be expected to be taken by the subject. This may be used to provide information about the type of medication in the absence of information about the medication actually taken by the subject. Prescription information may alternatively be used in combination with information about the medication actually taken by the subject to determine whether a change to the prescription should be recommended, or whether the subject is taking their medication as prescribed.

In some examples, the SDB therapy information is acquired by at least a pressure sensor and/or a flow sensor provided in the PAP device.

The inventors have recognized that sensors already included in a PAP device are capable of acquiring data that may be used to determine a measure of the efficacy and/or adherence of SDB therapy.

The SDB therapy information may additionally or alternatively be acquired by one or more of: a pulse oximetry sensor, an electrocardiography sensor, an electromyography sensor, an electroencephalography sensor, an accelerometer, an inertial measurement unit, and/or a position sensor.

In some examples, the at least one measure of therapy outcome comprises a loop gain; and the loop gain is determined by: controlling the PAP device to change the pressure at which air is provided to the subject; obtaining a signal responsive to a change in one or more physiological parameters; processing the signal to detect a change in the one or more physiological parameters; and determining a time between the change in pressure and the detected change in the one or more physiological parameters to determine the loop gain.

The loop gain is the time taken by the subject to adapt to a new breathing pattern when a difficulty of respiration changes. The loop gain provides a useful measure of SDB therapy efficacy, as a faster loop gain results in, for instance, a smaller decrease in oxygen saturation, and therefore reduces a likelihood that the subject's sleep is disturbed by an increase in respiration difficulty. Medication and PAP therapy can each affect the loop gain of a subject.

In some examples, the at least one type of medication taken by the subject comprises two or more types of medication, and the medication information comprises at least a type of medication taken for each of the two or more types of medication.

In some cases, a combination of medications may have a different effect on the SDB therapy than any/either one of the medications alone.

In some examples, in which the processing system is configured to control an output user interface to provide a user-perceptible output in response to the first predetermined condition being satisfied, in response to a third predetermined condition being satisfied, the processing system is further configured to: obtain, from a database of known interactions between medications, information relating to an interaction between the two or more types of medication; process the information relating to an interaction between the two or more types of medication to determine whether or not the first predetermined condition is satisfied.

In other words, known interactions between medications taken by the subject may be used, in some cases (e.g. borderline situations), to determine whether or not to provide a user-perceptible output representative of the correlation.

In some examples, the at least one type of medication taken by the subject comprises a medication for treating sleep disordered breathing.

In other words, the SDB therapy is combination therapy, in which PAP therapy is used in combination with pharmacotherapy.

According to another aspect of the invention, there is provided a computer-implemented method for determining a correlation between medication intake and sleep disordered breathing, SDB, therapy outcome for a subject taking at least one type of medication and being provided with SDB therapy comprising use of a positive airway pressure, PAP, device, the method comprising: obtaining medication information relating to a medication intake of the subject, wherein the medication information is indicative of at least a type of medication taken; obtaining SDB therapy information relating to SDB therapy provided to the subject during at least one sleep session, wherein the SDB therapy information comprises at least one measure of therapy outcome; and processing the medication information and the SDB therapy information to determine a correlation between the medication data and the SDB therapy information.

There is also provided a computer program product comprising computer program code means which, when executed on a computing device having a processing system, cause the processing system to perform all of the steps of the method described above.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:

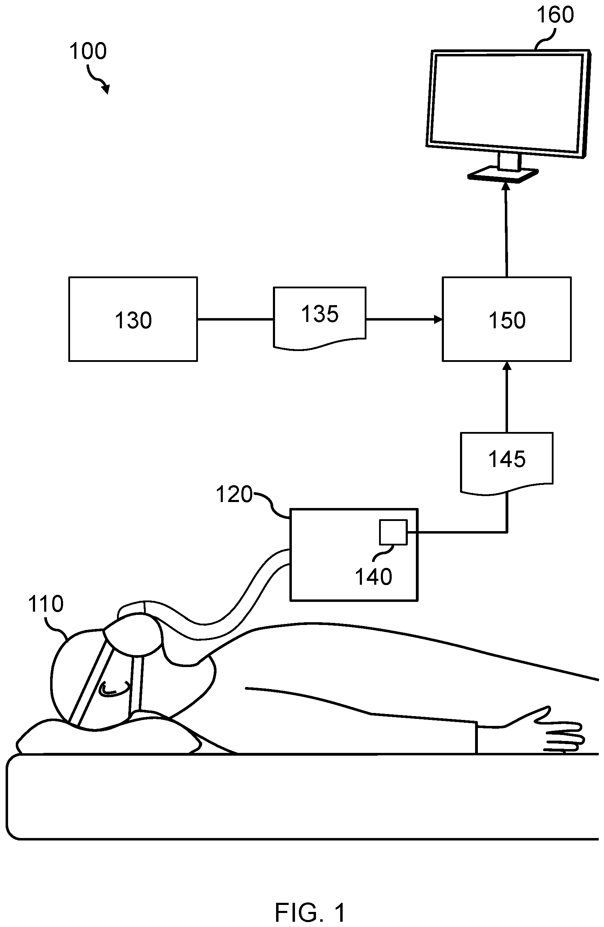

Fig. 1 illustrates a system for determining a correlation between medication intake and a sleep disordered breathing, SDB, therapy outcome for a subject taking at least one type of medication and being provided with SDB therapy comprising use of a positive airway pressure, PAP, device, according to an embodiment of the invention; and

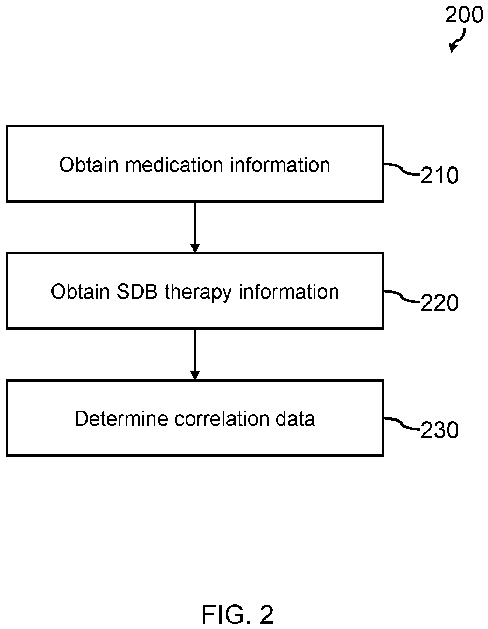

Fig. 2 illustrates a computer-implemented method for determining a correlation between medication intake and an SDB therapy output for a subject taking at least one type of medication and being provided with SDB therapy comprising use of a PAP device, according to an embodiment of the invention.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a system and method for determining a correlation between medication intake and sleep disordered breathing, SDB, therapy outcome for a subject taking at least one type of medication and being provided with SDB therapy. The SDB therapy comprises at least positive airway pressure, PAP, therapy provided by a PAP device. Medication information, relating to a medication intake of the subject, and SDB therapy information, relating to a therapy outcome of the SDB therapy, are obtained and correlated.

Embodiments are at least partly based on the realization that an interaction between a medication (or combination of medications) and SDB therapy outcome may be identified (and thus, if required, addressed) by correlating medication information and SDB therapy information.

Illustrative embodiments may, for example, be employed in PAP therapy systems, sleep monitoring systems, and patient adherence management systems.

Fig. 1 illustrates a system 100 for determining a correlation between medication intake and a sleep disordered breathing, SDB, therapy outcome for a subject 110 taking at least one type of medication and being provided with SDB therapy comprising use of a positive airway pressure, PAP, device 120, according to an embodiment of the invention.

The PAP device 120 used to provide the SDB therapy may be any type of PAP device (e.g. a CPAP device, BiPAP device or APAP device), and comprises a PAP machine that generates pressurized air, a mask that fits over the subject's nose and/or mouth, and a tube connecting the mask to the PAP machine.

The system 100 comprises a medication information unit 130, one or more sensors 140, and a processing system 150. The processing system is, itself, an embodiment of the invention.

The medication information unit 130 is configured to acquire medication information 135 relating to a medication intake of the subject 110. The medication information is indicative of at least a type of medication taken by the subject. In some examples, the medication information may further comprise additional information, such as a dosage of medication, a timing of taking medication for at least one medication taken by the subject (e.g. a time of day, a time relative to a sleep session of the subject and/or a time relative to a meal of the subject, e.g. whether the medication intake is before or after the meal, and optionally how long before or after the meal) and/or a form of administration (e.g. in tablet form, intravenously, etc.).

In some examples, where the subject is taking two or more types of medication, the medication information 135 may comprise at least a type of medication for each of the two or more types of medication taken by the subject. In some examples, the subject may take at least one type of medication for treating sleep disordered breathing.

Preferably, the medication information unit 130 comprises a unit for obtaining information about medication actually taken by the subject, such as a user input device, a sensor of a medication-dispensing device (e.g. a sensor of a smart pill dispenser, medication pump, nebulizer or arterial line), a camera and/or a wearable biosensor. For instance, the subject 110 may input their medication intake into a user input device.

Techniques for using a sensor of a medication-dispensing device, a camera and/or a wearable biosensor to acquire medication information will be apparent to the skilled person. See, for example: Abbey et al. (2012), "A remotely programmable smart pillbox for enhancing medication adherence", 2012 25th IEEE International Symposium on Computer-Based Medical Systems (CBMS); DeMeo and Morena (2014), "Medication adherence using a smart pill bottle", 2014 11th International Conference & Expo on Emerging Technologies for a Smarter World (CEWIT); Bilodeau and Ammouri (2011), "Monitoring of medication intake using a camera system", J Med Syst 35:377-389; and Rumbut, J. et al. (2020), "Harmonizing wearable biosensor data streams to test polysubstance detection", Int Conf Comput. Netw. Commun., February 2020:445-449.

Additionally or alternatively, the medication information unit 130 may obtain prescription information relating to a type of medication prescribed to the subject, and optionally to a prescribed dosage, timing and/or form of administration. For example, the medication information unit may be an electronic medical record (EMR) or electronic health record (EHR) database. Prescription information may be used in the absence of information about medication actually taken by the subject, or may be used in combination with information about medication actually taken by the subject (for instance, in order to determine whether an adjustment should be made to the prescription).

The one or more sensors 140 are configured to acquire SDB therapy information 145 relating to SDB therapy provided to the subject during at least one sleep session. The SDB therapy information comprises at least one measure of therapy outcome. The at least one measure of therapy outcome may comprise a measure of therapy efficacy and/or a measure of therapy adherence.

Preferably, the SDB therapy information 145 relates to a plurality of sleep sessions. The plurality of sleep sessions may include at least one sleep session for which no medication is taken, and/or sleep sessions for which the medication intake of the subject is different (e.g. medication is taken at a different time, a different dosage of medication is taken, and/or a different type of medication is taken).

For instance, when introducing a new type of medication, SDB therapy information may be obtained for one or more first sleep sessions during which the new medication is not taken (e.g. for a week) and for one or more second sleep sessions during which the new medication is taken at a first dosage, and optionally, for one or more third sleep sessions during which the new medication is taken at a second dosage.

In some examples, where the medication taken by the subject is slow-acting, SDB therapy information for sleep sessions for which the subject is taking the medication may be obtained once the subject has taken the medication for long enough to take effect. Similarly, in order to obtain SDB therapy information for sleep sessions for which the subject has taken no medication, the subject may be requested to refrain from taking medication for long enough to allow the effect of the medication to decay. In some examples, the processing system may determine a time needed for the effect of a medication taken by the subject to decay based on a known typical decay curve of the medication.

In some examples, a single sleep session (or a plurality of sleep sessions with identical or similar medication intakes) may be sufficient to determine useful correlation data. For instance, if the medication taken by the subject comprises medication that decays quickly (i.e. quickly relative to the length of the sleep session), such as nasal decongestants and diabetes drugs, SDB therapy information acquired at different times during the sleep session(s) as the effect of the medication wears off may be used to determine a correlation between the medication and the SDB therapy outcome.

The SDB therapy provided to the subject during the at least one sleep session comprises positive airway pressure (PAP) therapy provided by the PAP device 120. In some examples, the SDB therapy may consist only of PAP therapy, while in other examples, the SDB therapy may comprise PAP therapy in combination with one or more other types of SDB therapy (e.g. pharmacotherapy, positional therapy and/or mandibular advancement therapy). For instance, the SDB therapy may further comprise at least one medication taken by the subject.

The at least one measure of therapy outcome may be any measure suitable for assessing an efficacy of and/or adherence to the SDB therapy prescribed to the subject. For instance, the at least one measure of therapy outcome may comprise one or more of: an apnea-hypopnea index (AHI), an oxygen desaturation index (ODI), a respiratory disturbance index (RDI), an oxygen saturation level, a number of SDB events, an average duration of SDB events, a type of SDB event, a respiration rate, a respiration depth, a number and/or frequency of arousals, a duration of one or more sleep stages, a proportion of a sleep session spent in a sleep stage, a frequency and/or duration of snoring, a measure of usage of the PAP device (e.g. number of hours the PAP device is used in a sleep session), and/or a subjective user assessment of therapy efficacy and/or adherence (e.g. a subjective user assessment of the subject's daytime sleepiness, comfort using the PAP device and/or adherence to the PAP therapy).

The at least one measure of therapy outcome may further comprise one or more of: a loop gain, a cardiac parameter (e.g. a heart rate, a heart rate variability, a measure of cardiac arrhythmia, a hemodynamic parameter, a blood pressure etc.), a measure of sympathetic activity, a measure of muscle activity, a measure of brain activity, an exhaled CO₂ concentration, a body temperature, a measure of sweating, a measure of bioimpedance, a measure of daytime physical activity, a measure of cognitive function, a body position during the at least one sleep session, a type of body movement during the at least one sleep session (e.g. tremor, restless leg, etc.) and/or a measure of mask leakage.

In some examples, the SDB therapy information 145 may further comprise timing information relating to a time at which a value of a measure of therapy outcome was measured, in order to assess the effect of a timing of medication intake on the SDB therapy outcome.

In Fig. 1, the one or more sensors 140 comprise a pressure sensor and/or a flow sensor in the PAP device 120. The inventors have advantageously recognized that these sensors are typically provided within existing PAP devices, and that such sensors can provide a measure of therapy outcome.

In some examples, the one or more sensors 140 may additionally or alternatively include other sensors capable of acquiring SDB therapy information. For instance, the one or more sensors may additionally or alternatively comprise one or more of: a pulse oximetry sensor, a microphone, an electrocardiography sensor, an electromyography sensor, an electroencephalography sensor, an accelerometer, an inertial measurement unit and/or a position sensor. Further examples of suitable sensors will be apparent to the skilled person. As the skilled person will readily appreciate, the type(s) of sensor used to acquire SDB therapy information may depend on a type of medication taken by the subject; for instance, a cardiac sensor may be used in the case of a subject who has been prescribed medication that is known to interact with the heart.

The one or more sensors may, as appropriate, be provided in the PAP device 120 (e.g. in the PAP machine and/or in the mask), in a wearable device worn by the subject 110 (e.g. a smartwatch, a chest belt, an abdominal belt, a snore relief band, a positional therapy device, etc.), and/or a nearable device (e.g. a smartphone).

Techniques for using one or more of the above-mentioned sensors to acquire SDB therapy information will be apparent to the skilled person. See, for example: Nakano et al. (2007), "Automatic detection of sleep-disordered breathing from a single-channel airflow record", European Respiratory Journal, 29:728-736; Haidar et al. (2017), "Sleep apnea event detection from nasal airflow using convolutional neural networks", ICONIP 2017: Neural Information Processing, pp. 819-827; Yang et al. (2019), "Sleep apnea and hypopnea events detection based on airflow signals using LSTM network", 2019 41st Annual International Conference of the IEEE Engineering in Medicine and Biology Society (EMBC), pp. 2576-2579; Koley and Dey (2013), "Real-time adaptive apnea and hypopnea event detection methodology for portable sleep apnea monitoring devices", IEEE Transactions on Biomedical Engineering, 60(12):3354-3363; John et al. (2021), "SomnNET: An SpO2 based deep learning network for sleep apnea detection in smartwatches", 2021 43rd Annual International Conference of the IEEE Engineering in Medicine & Biology Society, pp. 1961-1964; and Nam et al. (2016), "Sleep monitoring based on a tri-axial accelerometer and a pressure sensor", Sensors, 16(5):750.

A loop gain (i.e. a time taken for the subject to respond physiologically to a change in respiration difficulty) may be determined by controlling the PAP device 120 to change the pressure at which air is provided to the subject, and obtaining a signal responsive to a change in one or more physiological parameters. The signal responsive to a change in one or more physiological parameters may be measured by the one or more sensors 140, and may, for example, comprise a signal responsive to one or more of: heart rate, respiration rate, respiration depth and/or muscle activity. The signal may then be processed to detect change in the one or more physiological parameters, and the loop gain may be determined as a time between the change in pressure provided by the PAP device and the detected change in the one or more physiological parameters.

The processing system 150 is configured to obtain the medication information 135 and the SDB therapy information 145, and to process the medication information and the SDB therapy information to determine correlation data indicating a correlation between the medication information and the SDB therapy information.

Methods of correlating data are well-known, and suitable methods for determining correlation data indicating a correlation between the medication information and the SDB information, such as cross-correlation and regression, will be apparent to the skilled person. In some examples, machine-learning techniques may be used to determine the correlation data.

The correlation data may comprise an indication of whether there is a non-zero correlation between the medication information 135 and the SDB therapy information 145, and may comprise an indication of whether a non-zero correlation is positive or negative. In some examples, the correlation data may comprise a measure of a strength of the correlation (e.g. a Pearson coefficient). The correlation data may further comprise a time lag of the correlation (where the medication information includes a timing of medication intake).

In some examples, in response to the correlation data and/or the SDB therapy information 145 satisfying a first predetermined condition, the processing system 150 is configured to control an output user interface 160 to provide a user-perceptible output representative of the correlation data.

Suitable user-perceptible outputs will be apparent to the skilled person. For instance, the user-perceptible output may comprise a textual display (e.g. stating that a medication or combination of medications has a positive, negative or neutral effect on SDB therapy outcome, providing a value of the Pearson coefficient, etc.), a color (e.g. a first color may indicate that the correlation is positive and a second color may indicate that the correlation is negative, and/or a shade of color may indicate a strength of the correlation), and/or an image (e.g. a correlation chart or matrix).

In Fig. 1, the output user interface 160 is a computer monitor; however, the skilled person will readily appreciate that any output user interface capable of providing a suitable user-perceptible output may be used (e.g. a mobile device or a speaker). The user-perceptible output may be provided to a clinician or caregiver of the subject, or to the subject; which user is provided with the user-perceptible output may depend on the content of the user-perceptible output.

The first predetermined condition used to determine whether a user-perceptible output of the correlation is provided may relate to the correlation data. For example, the first predetermined condition may be satisfied in response to a determination that a correlation between the medication information 135 and the SDB therapy information 145 is negative and/or in response to a strength of the correlation exceeding a predetermined correlation threshold.

In other examples, the first predetermined condition may additionally or alternatively relate to the SDB therapy information 145. In particular, a user-perceptible output may be provided in response to a determination of a negative therapy outcome. For example, the first predetermined condition may be satisfied in response to a determination that the at least one measure of therapy outcome breaches a predetermined therapy outcome threshold, or that a change in the at least one measure of therapy outcome correlated with medication intake breaches a predetermined effect threshold. For instance, the first predetermined condition may relate to an increase in the number and/or severity of SDB events above a predetermined percentage in response to a change in medication intake.

In some examples, the first predetermined condition may be defined by the subject's clinician.

In some examples, the determination as to whether a user-perceptible output of the correlation is provided may, where the medication information indicates that the subject has taken two or more types of medication, take into account known interactions between the two or more types of medications. For instance, information relating to an interaction between the two or more types of medication may be obtained in response to a third predetermined condition being satisfied.

The third predetermined condition may relate to "borderline" cases: for instance, where the first predetermined condition comprises a threshold for a value (e.g. a correlation strength, a change in the at least one measure of therapy outcome, etc.), the third predetermined condition may be satisfied in response to the value falling within a predetermined margin of the threshold. In another example, the third predetermined condition may be satisfied in response to a failure to satisfy the first predetermined condition.

Information relating to an interaction between the two or more types of medication may be obtained from one or more databases of known interactions between medications (such as the databases provided by DDInter, Medscape and Drugs.com). The information relating to an interaction between the two or more types of medication may comprise an interaction between each pair of medications in the two or more types of medication, and, where available, an interaction between all of the medication in the two of more types of medication.

A user-perceptible output of the correlation may be provided in response to the information relating to an interaction between the two or more types of medication indicating a negative interaction.

In some examples, the processing system 150 may additionally or alternatively process the correlation data to determine a first recommended action. The first recommended action may be an action to improve SDB therapy outcome. For instance, the first recommended action may be an action to increase an effect of medication intake on SDB therapy outcome, where the correlation data indicates that the medication intake has a positive effect on therapy outcome (e.g. where the medication taken by the subject is medication for treating SDB), or to decrease an effect of medication intake on SDB therapy outcome or compensate for the effect of medication, where the correlation data indicates that the medication intake has a negative effect on therapy outcome. The processing system may use a set of predetermined rules to determine the first recommended action.

The first recommended action may comprise a change in medication intake (e.g. a change in a type, dosage and/or timing of medication), and/or a change in the PAP therapy provided to the subject (e.g. a change in a pressure of air provided by the PAP device, a change in the prescribed hours of use of the PAP device, a change of PAP therapy modality, and/or a change to combination therapy).

For instance, hypnotics may increase AHI due to pharyngeal muscle relaxation and cause delayed arousals due to worsening hypoxemia. The processing system 150 may therefore, in response to detecting a correlation between an intake of a hypnotic medication and AHI and/or between an intake of a hypnotic medication and arousal latency, recommend increasing the air pressure provided by the PAP device 120.

In another example, anxiety medication may result in longer sleep and an increase in drowsiness. The processing system 150 may therefore, in response to detecting a correlation between an intake of an anxiety medication and daytime sleepiness, recommend reducing the number of hours for which the PAP device 120 used in a sleep session.

In yet another example, hypnotics and anxiety medication may each alter the sleep stages experienced by the subject during a sleep session, increasing an amount of N3 and REM sleep. This may reduce PAP therapy adherence, as many subjects remove their mask during periods of sleep in which REM is more prominent. The processing system 150 may therefore, in response to detecting a correlation between an intake of a hypnotic medication and/or an anxiety medication and a frequency and/or duration of N3 or REM sleep, recommend a change of PAP modality to auto-BiPAP therapy and/or recommend the use of PAP therapy in combination with an alternative SDB therapy, such as positional therapy and/or mandibular advancement therapy.

In yet another example, the effect of a medication on SDB therapy outcome may depend on a timing of medication intake. The processing system 150 may therefore, in response to detecting a correlation between a timing of medication intake and the at least one measure of therapy outcome, and determining that a current timing of medication intake negatively affects the measure of therapy outcome, recommend adjusting a timing of medication intake.

As the skilled person will readily appreciate, a suitable action to recommend may depend not only on the correlation between medication intake and therapy outcome, but also on additional factors including the type(s) of medication taken by the subject, a current air pressure and PAP modality of the PAP device, and/or a phenotype of the subject. In some examples, the processing system may additionally process one or more of these additional factors when determining the first recommended action.

For example, the processing system 150 may recommend a change in PAP therapy rather than a change in medication intake if it is determined that the medication taken by the subject cannot be changed. The processing system may recommend a change in medication intake or a change to combination therapy (i.e. PAP therapy in combination with one or more other forms of SDB therapy) if it is determined that any increase in the pressure provided by the PAP device would result in an excessively high pressure.

In some cases, the recommended action may be different for different phenotypes. For instance, the processing system 150 may further obtain phenotype information for the subject, and may additionally process the phenotype information to determine the first recommended action. The phenotype information may comprise one or more of: a gender, an age, a body weight, and/or a body mass index.

In some examples, the processing system 150 may iteratively modify a recommended action until a desired effect (e.g. a desired therapy outcome) is achieved. For example, the processing system may be configured to monitor an effect of the first recommended action, by continuing to obtain medication information 135 and SDB therapy information 145 for the subject after the first recommended action has been implemented, and processing the medication information and SDB therapy information to determine an effect of the first recommended action. In response to the effect of the first recommended action satisfying a second predetermined condition, the processing system may process the effect of the first recommended action to determine a second recommended action. The processing system may then monitor the effect of the second recommended action, and so on, until the second predetermined condition is satisfied.

The second predetermined condition may, for example, relate to a change in the SDB therapy information after the first recommended action has been implemented. For instance, the second predetermined condition may comprise a threshold for at least one measure of therapy outcome (e.g. an AHI, ODI or RDI threshold).

The second recommended action may depend on a type of effect of the first recommended action. For instance, in response to a determination that the first recommended action has a positive, but insufficient, effect on the SDB therapy outcome, the processing system 150 may recommended an additional action to further improve SDB therapy outcome. In response to a determination that the first recommended action has a negative effect on SDB therapy outcome, the processing system may recommend an action to reverse or counteract the effect of the first recommended action.

In some examples, the processing system 150 may follow a predetermined order of preference for recommended actions when determining the first recommended action, second recommended action, and so on. For instance, the processing system may be configured to first recommend one or more actions relating to the PAP therapy provided to the subject (e.g. adjusting the air pressure provided, changing the PAP modality from CPAP to BiPAP or Auto-PAP, etc.), and only recommend an action relating to an adjustment to medication if actions relating to the PAP therapy fail to provide a desired effect. The predetermined order may, for example, depend on the medication intake of the subject. In some examples, the predetermined order may be subject-specific (e.g. determined by the subject's clinician)/.

In some examples, the processing system 150 may be configured to control an output user interface to provide a user-perceptible output of the recommended action, e.g. a textual display of the recommended action, so that the recommended action may be implemented. Depending on the content of the recommended action, the user-perceptible output may be provided to the subject (e.g. for minor adjustments) or to a clinician. The processing system may follow a predetermined set of rules to decide whether to output the recommended action to the subject or to a clinician.

In some examples, where the recommended action comprises an adjusted pressure to be provided by the PAP device 120, the processing system 150 may be configured to generate a control signal configured to control the PAP device to provide air to the subject at the adjusted pressure.

Fig. 2 illustrates a computer-implemented method 200 for determining a correlation between medication intake and an SDB therapy outcome for a subject taking at least one type of medication and being provided with SDB therapy comprising use of a PAP device, according to an embodiment of the invention.

The method 200 begins at step 210, at which medication information relating to a medication intake of the subject is obtained. The medication information is indicative of at least a type of medication taken by the subject.

At step 220, SDB therapy information relating to SDB therapy provided to the subject during at least one sleep session is obtained. The SDB therapy information comprises at least one measure of therapy outcome.

At step 230, the medication information and SDB therapy information are processed to determine correlation data indicating a correlation between the medication information and the SDB therapy information.

It will be understood that the disclosed methods are computer-implemented methods. As such, there is also proposed a concept of a computer program comprising code means for implementing any described method when said program is run on a processing system.

The skilled person would be readily capable of developing a processing system for carrying out any herein described method. Thus, each step of a flow chart may represent a different action performed by a processing system, and may be performed by a respective module of the processing system.

As discussed above, the system makes use of a processing system to perform the data processing. The processing system can be implemented in numerous ways, with software and/or hardware, to perform the various functions required. The processing system typically employs one or more microprocessors that may be programmed using software (e.g. microcode) to perform the required functions. The processing system may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs). Thus, the processing system may be embodied as a digital and/or analog processing system.

In various implementations, the processing system may be associated with one or more storage media such as volatile and non-volatile computer memory such as RAM, PROM, EPROM, and EEPROM. The storage media may be encoded with one or more programs that, when executed on one or more processing systems and/or controllers, perform the required functions. Various storage media may be fixed within a processing system or controller may be transportable, such that the one or more programs stored thereon can be loaded into a processing system.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

Functions implemented by a processor may be implemented by a single processor or by multiple separate processing units which may together be considered to constitute a "processor". Such processing units may in some cases be remote from each other and communicate with each other in a wired or wireless manner.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.

Any reference signs in the claims should not be construed as limiting the scope.

## Claims

1. A processing system (150) for determining a correlation between medication intake and sleep disordered breathing, SDB, therapy outcome for a subject (110) taking at least one type of medication and being provided with SDB therapy comprising use of a positive airway pressure, PAP, device (120), the processing system being configured to:
obtain medication information (135) relating to a medication intake of the subject, wherein the medication information is indicative of at least a type of medication taken;
obtain SDB therapy information (145) relating to SDB therapy provided to the subject during at least one sleep session, wherein the SDB therapy information comprises at least one measure of therapy outcome; and
process the medication information and the SDB therapy information to determine correlation data indicating a correlation between the medication data and the SDB therapy information.

2. The processing system (150) of claim 1, wherein, in response to the correlation data and/or the SDB therapy information (145) satisfying a first predetermined condition, the processing system is further configured to control an output user interface (160) to provide a user-perceptible output representative of the correlation data.

3. The processing system (150) of claim 1 or 2, wherein the processing system is further configured to process the correlation data to determine a first recommended action.

4. The processing system (150) of claim 3, wherein the processing system is further configured to control an output user interface to provide a user-perceptible output of the first recommended action.

5. The processing system (150) of claim 3 or 4, wherein:
the first recommended action comprises an adjusted pressure to be provided by the PAP device; and
the processing system is further configured to generate a control signal configured to control the PAP device to provide air to the subject at the adjusted pressure.

6. The processing system (150) of any of claims 3 to 5, wherein the processing system is further configured to:
continue to obtain medication information (135) and SDB therapy information (145) for the subject after the first recommended action has been implemented;
process the medication information and SDB therapy information to determine an effect of the first recommended action; and
in response to the effect of the first recommended action satisfying a second predetermined condition, process the effect of the first recommended action to determine a second recommended action.

7. The processing system (150) of any of claims 1 to 6, wherein the medication information (135) further comprises a dosage and/or a timing of taking medication for at least one medication taken by the subject.

8. The processing system (150) of any of claims 1 to 7, wherein the medication information (135) is acquired by at least one of: a user input device, a sensor of a medication-dispensing device, a camera and/or a wearable biosensor.

9. The processing system (150) of any of claims 1 to 8, wherein the SDB therapy information (145) is acquired by at least a pressure sensor and/or a flow sensor provided in the PAP device (120).

10. The processing system (150) of any of claims 1 to 9, wherein:
the at least one measure of therapy outcome comprises a loop gain; and
the loop gain is determined by:
controlling the PAP device (120) to change the pressure at which air is provided to the subject;
obtaining a signal responsive to a change in one or more physiological parameters;
processing the signal to detect a change in the one or more physiological parameters; and
determining a time between the change in pressure and the detected change in the one or more physiological parameters to determine the loop gain.

11. The processing system (150) of any of claims 1 to 10, wherein:
the at least one type of medication taken by the subject comprises two or more types of medication; and
the medication information (135) comprises at least a type of medication taken for each of the two or more types of medication.

12. The processing system (150) of claim 11, when dependent on claim 2, wherein, in response to a third predetermined condition being satisfied, the processing system is further configured to:
obtain, from a database of known interactions between medications, information relating to an interaction between the two or more types of medication;
process the information relating to an interaction between the two or more types of medication to determine whether or not the first predetermined condition is satisfied.

13. The processing system (150) of any of claims 1 to 12, wherein the at least one type of medication taken by the subject comprises a medication for treating sleep disordered breathing.

14. A computer-implemented method (200) for determining a correlation between medication intake and sleep disordered breathing, SDB, therapy outcome for a subject (110) taking at least one type of medication and being provided with SDB therapy comprising use of a positive airway pressure, PAP, device (120), the method comprising:
obtaining medication information (135) relating to a medication intake of the subject, wherein the medication information is indicative of at least a type of medication taken;
obtaining SDB therapy information (145) relating to SDB therapy provided to the subject during at least one sleep session, wherein the SDB therapy information comprises at least one measure of therapy outcome; and
processing the medication information and the SDB therapy information to determine a correlation between the medication data and the SDB therapy information.

15. A computer program product comprising computer program code means which, when executed on a computing device having a processing system, cause the processing system to perform all of the steps of the method (200) according to claim 14.
